# EUROPEAN PATENT APPLICATION

(11) **EP 0 997 120 A1**
(43) Date of publication of application: **03.05.2000**
(21) Application number: 99308439.1
(22) Date of filing: 26.10.1999
(51) Int. Cl.: A61F 5/08

(54) **Nose-shaping device**

(30) Priority: 30.10.1998 KR 9846473
(71) Applicant: Hong, Seong-Churl, Seongnam-City, Kyungki-Do (KR)
(72) Inventor: Hong, Seong-Churl, Seongnam-City, Kyungki-Do (KR)
(74) Representative: Neill, Alastair William

(57) **Abstract**

A nose-shaping device comprises a lifting cap which is inserted in the nostril and contacts with the ridge of the nose, a supporting cap which contacts with the rear surface in the nostril and supports the lifting cap, a connecting bar which connects the lifting cap to the supporting cap, the connecting bar being capable of expansion and contraction, and an elastic member which is disposed on the outer circumference of the connecting bar to apply the elastic force to the lifting cap so as to heighten the ridge of the nose, the both ends of the elastic member contacting with the lifting cap and the supporting cap, respectively.

## Description

### BACKGROUND OF THE INVENTION

### 1) Field of the invention

The present invention relates to a nose-shaping device and, more particularly, to a nose-shaping device which heightens the septal cartilage(the bridge of the nose) and the lateral cartilage (the ridge of the nose) and narrows the alar cartilage(the wings of the nose).

### 2) Description of Related Art

Recently, people are concerned about the beauty increasingly, there are many beauty products out in the market. There are cosmetic products of the eyes, lips, hair, and even for the breasts, but there have been actually no alternatives for plastic operation to improve the looks of the nose. In particular, most Asians who typically have flat nose bridge and round nostril are dissatisfied with their noses.

As described above, the general method for heightening the nose is an expensive and painstaking plastic operation to insert a foreign material like silicone into the bridge of the nose.

However, the plastic operation often brings side-effects like inflammation or pain, etc., and the whole face becomes misshapen in the worst case.

In addition, after the operation is performed, the outdoor life is restricted for a definite time, and other people become easily aware of the change in the look.

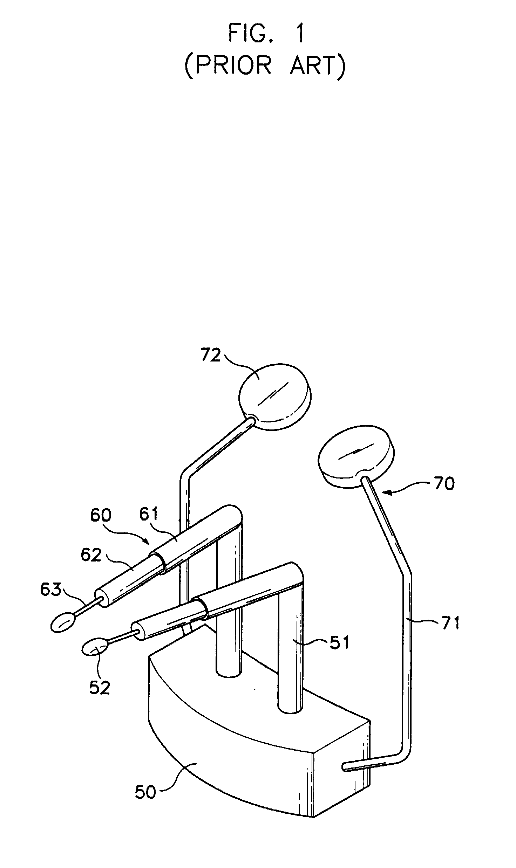

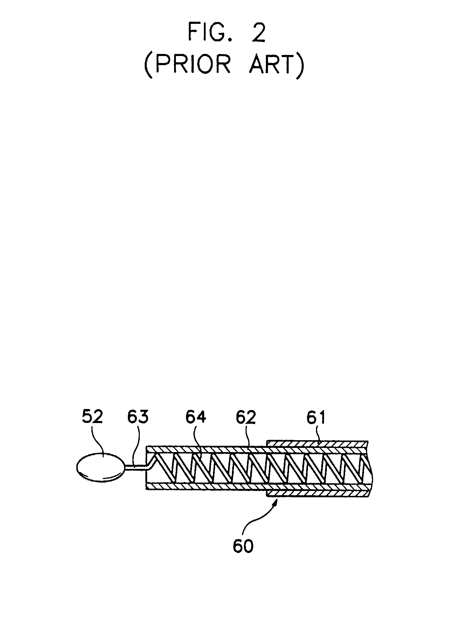

In the meantime, Korean laid-open patent No. 98-033691 discloses a nose-shaping device for preventing snoring and heightening the nose. This will be described hereinafter with reference to Figs. 1 and 2.

As shown in the drawings, in the conventional nose-shaping device, a pair of support bars 51 are mounted vertically on the rear portion of a housing 50, elastic means 60 are fixed on each front end of the support bars 51, ribs 63 are fixed on each front end of the elastic means 60, and inner pushing members 52 which are placed in the nostril are connected to each front end of the ribs 63. When the conventional nose-shaping device is put on, the housing 50 is placed between the nose and the upper lip.

And, an upper pushing means 70 comprises a pair of elastic bars 71 mounted vertically on each side portion of the housing 50, and upper pushing members 72 fixed on each front end of the elastic bars 71 for heightening the upper portion of the nose.

Each elastic means 60 includes an outer pipe 61 fixed on the support bar 51, an inner pipe 62 inserted into the outer pipe 61, and a spring 64 which is provided inside of the outer and inner pipes 61 and 62 and connected to the rib 63 so as to push the inner pushing member 52 toward the ridge of the nose by applying the elastic force.

However, when the user wears the conventional nose-shaping device, since the housing 50, the support bars 51, and the upper pushing means 70 are placed outside of the nose, i.e. on the face, so the user becomes conscious of other person's eyes and is considerably restricted in time and place in wearing the nose-shaping device. In addition, because the spring 64 for biasing the inner pushing member 52 is provided inside of the outer and inner pipes 61 and 62 and elastic means 60 is fixed integrally on the support bar 51 and the housing 50, it is impossible to adjust the length and the elastic force of the spring 64 to user's various desire and to renew only spring 64 when it operates abnormally. And, since the outer and inner pipes 61 and 62 should be formed larger than the spring 64, the whole size of the elastic means 60 becomes relatively large, thereby deteriorating a feeling of wear.

### SUMMARY OF THE INVENTION

Therefore, the present invention has been made in an effort to solve the above-described problems.

It is an objective of the present invention to provide a nose-shaping device which is invisible from outside to keep user's privacy, so that the user can wear conveniently and freely from the restriction of time and place and other person's eyes.

It is another objective of the present invention to provide a nose-shaping device which can be adjusted easily to user's desire in size.

To achieve above objectives, the present invention provides a nose-shaping device comprising a lifting cap which is inserted in the nostril and contacts with the ridge of the nose, a supporting cap which contacts with the rear surface in the nostril and supports the lifting cap, a connecting bar which connects the lifting cap to the supporting cap, the connecting bar being capable of expansion and contraction, and an elastic member which is disposed on the outer circumference of the connecting bar to apply the elastic force to the lifting cap so as to heighten the ridge of the nose, the both ends of the elastic member contacting with the lifting cap and the supporting cap, respectively.

### BRIEF DESCRIPTION OF THE DRAWINGS

The accompanying drawings, which are incorporated in and constitute a part of the specification, illustrate an embodiment of the invention, and together with the description, serve to explain the principles of the invention:
Fig. 1 is a perspective view of a conventional nose-shaping device;
Fig. 2 is a sectional view illustrating a principal part of a conventional nose-shaping device;
Fig. 3 is a perspective view of a nose-shaping device according to a preferred embodiment of the present invention;
Fig. 4 is an exploded perspective view of a nose-shaping device according to a preferred embodiment of the present invention;
Fig. 5a is a sectional view illustrating a contracted state of a nose-shaping device according to a preferred embodiment of the present invention;
Fig. 5b is a sectional view illustrating an expanded state of a nose-shaping device according to a preferred embodiment of the present invention;
Fig. 6 is a sectional view illustrating a state of placing a nose-shaping device according to a preferred embodiment of the present invention inside of nose.

### DETAILED DESCRIPTION OF THE PREFERRED EMBODIMENTS

A preferred embodiment of the present invention will now be described in detail with reference to the accompanying drawings.

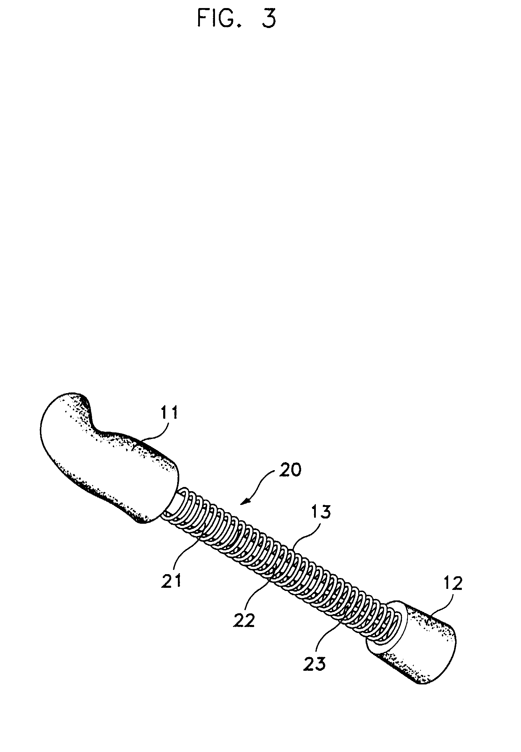

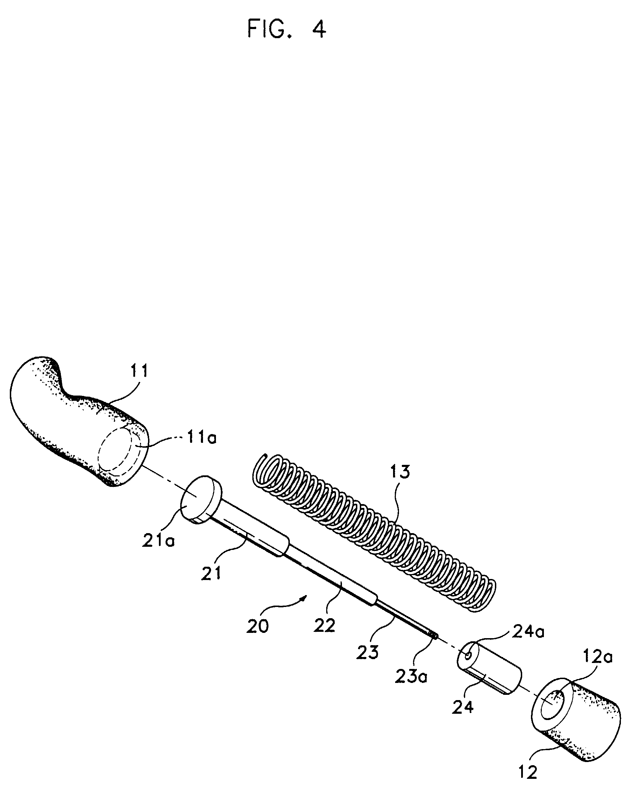

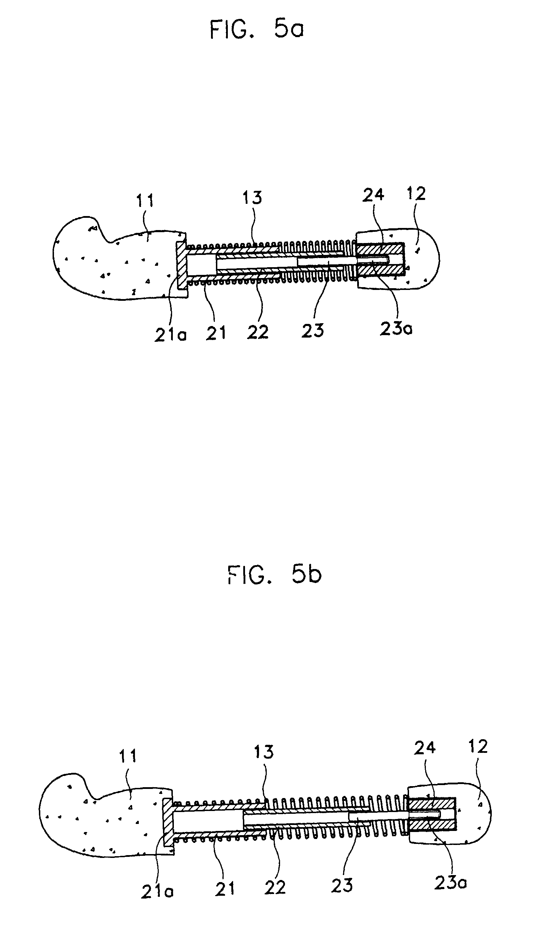

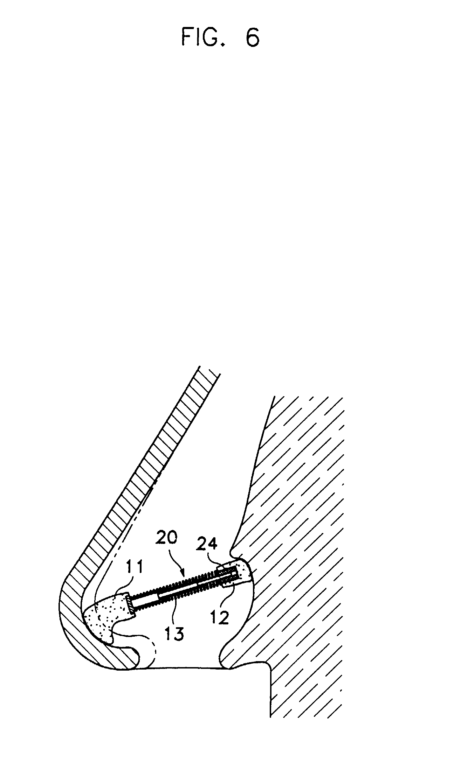

Figs. 3 and 4 are, respectively, a perspective view and an exploded perspective view of a nose-shaping device according to a preferred embodiment of the present invention, Figs. 5a and 5b are, respectively, are sectional views illustrating a contracted state and an expanded state of a nose-shaping device, and Fig. 6 is a sectional view illustrating a state of placing a nose-shaping device inside of nose.

As shown in the drawings, a nose-shaping device of the present invention comprises a lifting cap 11 which is inserted in the nostril and contacts with the ridge of the nose, a supporting cap 12 which contacts with the rear surface in the nostril and supports the lifting cap 11, a connecting bar 20 which connects the lifting cap 11 to the supporting cap 12, the connecting bar 20 being capable of expansion and contraction, and an elastic member 13 which is disposed on the outer circumference of the connecting bar 20 to apply the elastic force to the lifting cap 11 so as to heighten the ridge of the nose, the both ends of the elastic member 13 contacting with the lifting cap 11 and the supporting cap 12, respectively.

The connecting bar 20 includes three bars 21, 22, 23 which have different diameters from each other. The third bar 23 is inserted slidably into the second bar 22, and the second bar 22 is inserted slidably into the first bar 21. Such a connecting bar 20 can be expanded to a predetermined length by the elastic member 13 disposed on the outer circumference thereof, which will be described later.

On one end of the first bar 21 is formed integrally a circular coupling plate 21a whose diameter is larger than that of the first bar 21. The coupling plate 21a is inserted fixedly into a first coupling hole lla formed on the lifting cap 11 and supports one end of the elastic member 13. And, on one end of the third bar 23 is formed a screw 23a which is coupled detachably to a coupling bar 24 mentioned hereinafter.

The coupling bar 24 is inserted fixedly into a second coupling hole 12a formed on the supporting cap 12, and has a screw hole 24a to which the screw 23a of the third bar 23 is coupled detachably.

Preferably, the lifting cap 11 and the supporting cap 12 are made of medical anti-biotic silicone which has an excellent elasticity and a smooth feel, thus the flesh can be maintained sanitary without injury. Also, the lifting cap 11 has a curved form so as to fit any shape of the nose and push the ridge of the nose smoothly in the nostril. In addition, when the user wears the inventive nose-shaping device, it is invisible from outside by inserted fully in the nostril, so the user's privacy can be kept.

The assembly structure and operation of the nose-shaping device according to an preferred embodiment of the present invention will be described hereinafter.

First, the coupling plate 21a formed on the connecting bar 20 is inserted fixedly into the first coupling hole 11a of the lifting cap 11, and the coupling bar 24 is inserted fixedly into the second coupling hole 12a of the supporting cap 12. Secondly, the elastic member 13 is disposed on the outer circumference of the connecting bar 20, and the screw 23a formed on the third bar 23 is coupled to the screw hole 24a of the coupling bar 24, thereby the assembly process of the nose-shaping device being completed. At this time, the both ends of the elastic member 13 are supported by the coupling plate 21a and the supporting cap 12, respectively, and the connecting bar 20 can be contracted and expanded to a predetermined length by the elastic member 13 as shown in Figs. 5a and 5b. Preferably, the elastic member 13 is a spring made of stainless steel.

In the meantime, since the size of the nose and the desire of heightening the nose to what extent are considerably various depending on the individual, the length of the nose-lifer should be adjusted conveniently. In the nose-shaping device of the present invention, if the spring 13 is not suitable for user, after pulling the spring 13 out of the connecting bar 20 by turning the screw 23a, the user can renew spring which is smaller or larger than old one, or shorten the spring 13 by cutting it with a kind of nail clipper, so that the length of the nose-shaping device can be adjusted easily to user's desire. Preferably, the length of the nose-shaping device is stretched from 13mm to 32mm to fit any size of the nose.

If the user places the nose-shaping device of the present invention in the nostril with squeezing the spring 13 with a kind of tweezers and releases the spring 13 to stretch out in a proper position as shown in Fig. 6, the lifting cap 11 heightens the septal cartilage(the bridge of the nose) and the lateral cartilage(the ridge of the nose) by the elastic force of the spring 13 and, at the same time, narrows the alar cartilage(the wings of the nose), thereby heightening any nose to bring back the beauty and confidence to anyone in one's physical appearance. In addition, long use of the nose-shaping device may even lead to a gradual correction of the nose shape.

As described above in detail, since the nose-shaping device of the present invention is invisible from outside by inserted fully in the nostril, the user can wear the nose-shaping device conveniently and freely from the restriction of time and place and other person's eyes. That is, the inventive nose-shaping device can be used in various occasion such as a job interview, an important business meeting, or all kinds of ceremony, etc..

Also, the length of the spring can be easily adjusted to the size of the nose and user's desire of heightening the nose to what extent, and only spring can be renewed when it operates abnormally or it is lost, thus the use is considerably convenient.

And, since the whole size of the nose-shaping device is relatively small, it is easy to keep and cleanse, so it can be always maintained sanitary.

The reader's attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings), may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extend to any novel one, or any novel combination, of the features disclosed in this specification (including any accompanying claims, abstract and drawings), or to any novel one, or any novel combination, of the steps of any method or process so disclosed.

## Claims

1. A nose-shaping device comprising:
a lifting cap which is inserted in the nostril and contacts with the ridge of the nose;
a supporting cap which contacts with the rear surface in the nostril and supports the lifting cap;
a connecting bar which connects the lifting cap to the supporting cap, the connecting bar being capable of expansion and contraction; and
an elastic member which is disposed on the outer circumference of the connecting bar to apply the elastic force to the lifting cap so as to heighten the ridge of the nose, the both ends of the elastic member contacting with the lifting cap and the supporting cap, respectively.

2. The nose-shaping device of claim 1 wherein the connecting bar includes a first bar, a second bar, and a third bar which have different diameters from each other, the third bar being inserted slidably into the second bar, and the second bar being inserted slidably into the first bar.

3. The nose-shaping device of claim 2 wherein on one end of the first bar is formed integrally a coupling plate whose diameter is larger than that of the first bar, the coupling plate being inserted fixedly into the lifting cap and supporting one end of the elastic member, and on one end of the third bar is formed a screw.

4. The nose-shaping device of claim 3 wherein the nose-shaping device further comprises a coupling bar which is inserted fixedly into the supporting cap and to which the screw of the third bar is coupled detachably, so as to connect the connecting bar to the supporting cap.

5. The nose-shaping device of claim 1 wherein the lifting cap and the supporting cap are made of medical anti-biotic silicone.

6. The nose-shaping device of claim 1 wherein the elastic member is a spring made of stainless steel.
